(19) Europäisches Patentamt
European Patent Office
Office européen des brevets

(11) Veröffentlichungsnummer: **0 295 370 B1**

(12) **EUROPÄISCHE PATENTSCHRIFT**

(45) Veröffentlichungstag der Patentschrift: **11.08.93**

(51) Int. Cl.⁵: **C07D 239/38**, C07D 239/34, C07D 239/26, //C09K19/34

(21) Anmeldenummer: **88104520.7**

(22) Anmeldetag: **22.03.88**

(54) **Flüssigkristalline 5-Phenylpyrimidin-Derivate mit Sc- oder Sc(Stern)-Phase und Verfahren zu ihrer Herstellung.**

(30) Priorität: **24.03.87 DE 3709618**

(43) Veröffentlichungstag der Anmeldung:
**21.12.88 Patentblatt 88/51**

(45) Bekanntmachung des Hinweises auf die Patenterteilung:
**11.08.93 Patentblatt 93/32**

(84) Benannte Vertragsstaaten:
**AT BE CH DE ES FR GB IT LI NL SE**

(56) Entgegenhaltungen:
**DE-A- 3 500 897**
**DE-A- 3 515 374**

(73) Patentinhaber: **HOECHST AKTIENGESELLSCHAFT**

**W-6230 Frankfurt am Main 80(DE)**

(72) Erfinder: **Müller, Ingrid, Dr.**
**Am Pringstbrunnen 1**
**W-6238 Hofheim am Taunus(DE)**
Erfinder: **Dübal, Hans-Rolf, Dr.**
**Heuhohlweg 6**
**W-6240 Königstein/Taunus(DE)**
Erfinder: **Escher, Claus, Dr.**
**Amselweg 3**
**W-6109 Mühltal(DE)**
Erfinder: **Hemmerling, Wolfgang, Dr.**
**Billtalstrasse 32**
**W-6231 Sulzbach (Taunus)(DE)**
Erfinder: **Ohlendorf, Dieter,Dr.**
**Am Kühlen Grund 4**
**W-6237 Liederbach(DE)**
Erfinder: **Wingen, Rainer, Dr.**
**Rotkäppchenweg 10**
**W-6234 Hatterscheim am Main(DE)**

EP 0 295 370 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Flüssigkristalle haben in jüngerer Zeit Eingang in verschiedenste technische Gebiete gefunden, in denen elektrooptische und Anzeigevorrichtungs-Eigenschaften gefragt sind (z.B. in Uhren-, Taschenrechner- und Schreibmaschinenanzeigen). Diese Anzeigevorrichtungen beruhen auf den dielektrischen Ausrichtungseffekten in den nematischen, cholesterischen und/oder smektischen Phasen der flüssigkristallinen Verbindungen, wobei - verursacht durch die dielektrische Anisotropie - die molekulare Längsachse der Verbindungen eine bestimmte Ausrichtung in einem angelegten elektrischen Feld einnimmt. Die üblichen Schaltzeiten bei diesen Anzeigevorrichtungen sind für viele andere potentielle Anwendungsgebiete von Flüssigkristallen, die an sich für die Technik wegen ihrer einzigartigen Eigenschaften sehr vielversprechende chemische Verbindungen sind, eher zu lang. Dieser Nachteil macht sich insbesondere dann bemerkbar, wenn - was bei größeren Anzeigeelementflächen zwangsläufig der Fall ist - eine große Anzahl von Bildpunkten angesteuert werden muß, wodurch die Produktionskosten solcher, diese größeren Flächen enthaltenden Geräte wie Videogeräte, Oszillographen oder Fernseh-, Radar-, EDV- oder Schreibautomaten-Bildschirme zu hoch wären.

Neben den nematischen und cholesterischen Flüssigkristallen haben seit einigen wenigen Jahren in zunehmendem Maß auch für Praxisanwendungen ferroelektrische, smektische Flüssigkristall-Phasen an Bedeutung gewonnen.

Clark und Lagerwall konnten zeigen, daß die Verwendung solcher Flüssigkristallsysteme in sehr dünnen Zellen zu optoelektrischen Schalt- oder Anzeigeelementen führt, die im Vergleich zu den herkömmlichen TN ("twisted nematic")-Zellen um bis zu etwa einem Faktor 1000 schnellere Schaltzeiten haben (vgl. z.B. Lagerwall et al. "Ferroelectric Liquid Crystals for Displays", SID Symposium, October Meeting 1985, San Diego, Ca., USA). Aufgrund dieser und anderer günstiger Eigenschaften, z.B. der bistabilen Schaltmöglichkeit und des nahezu blickwinkelunabhängigen Kontrasts, sind ferroelektrische Flüssigkristalle grundsätzlich für die oben genannten Anwendungsgebiete, z.B. über eine Matrixansteuerung, gut geeignet.

Für die praktische Verwendung von ferroelektrischen Flüssigkristallen in optoelektrischen Anzeigen werden nun chiral geneigte, smektische Phasen, z.B. $S_C^*$-Phasen, benötigt (R.B. Meyer, L. Liébert, L. Strzelecki, P. Keller, J. Physique 36, L-69 (1975)), die über einen großen Temperaturbereich stabil sind.

Dieses Ziel kann man erreichen mit Verbindungen, die selbst chirale smektische Phasen, z.B. $S_C^*$-Phasen, ausbilden, oder aber indem man nicht-chirale, geneigte smektische Phasen ausbildende Verbindungen mit optisch aktiven Verbindungen dotiert (M. Brunet, C. Williams, Ann. Phys.3, 237 (1978)).

Es besteht daher ein Bedarf an Verbindungen, die smektische Phasen ausbilden und mit Hilfe derer Mischungen mit smektischen, insbesondere $S_c$- oder $S_C^*$-Phasen, hergestellt werden können.

Überraschenderweise wurde nun gefunden, daß die neuen Verbindungen der Formel (I) flüssigkristalline $S_c$- bzw. $S_C^*$-Phasen ausbilden.

Die Erfindung betrifft somit:

1. 5-Phenyl-pyrimidin-Derivate, die dadurch gekennzeichnet sind, daß in der Formel (I)

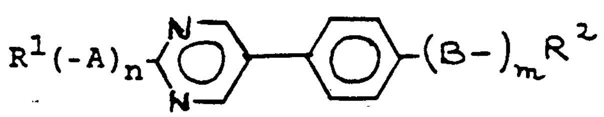
(I)

die Symbole folgende Bedeutung haben:

$R^1$ und $R^2$ bedeuten darin gleiche oder verschiedene, geradkettige oder verzweigte Alkyl- oder Alkenyl-Gruppen-mit k bis 16 C-Atomen, mit oder ohne asymmetrisches C-Atom, worin auch eine oder mehrere nicht benachbarte $-CH_2-$ Gruppen durch -O- und/oder -S- ersetzt sein können,

-A und -B bedeuten jeweils

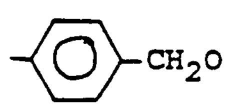
oder
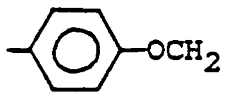

wobei jeweils die Reste $R^1$, $R^2$ am Phenylenteil von -A, -B sitzen
und

m und n bedeuten 0 oder 1, aber m und n nicht gleichzeitig 1, mit folgenden Maßgaben:

a) für m = n = 0 ist k = 8 und in $R^2$ ist die dem Phenylkern nächste $-CH_2$-Gruppe durch -O- ersetzt;

b) für m = 1 und n = 0 ist k = 2, und

c) für m = 0 und n = 1 ist k = 2 und in $R^2$ ist die dem Phenylkern nächste $-CH_2$-Gruppe durch -O- ersetzt.

2. Ein Verfahren zur Herstellung einer Verbindung der allgemeinen Formel (I), das dadurch gekennzeichnet ist, daß ein Trimethinsalz der Formel (VII) mit einem Amidiniumsalz der Formel (II) bzw. mit einem S-Alkylisothioharnstoffsalz der Formel (IV) in Gegenwart einer Base zu den Verbindungen (X) bzw. (VIII) umgesetzt wird und die freien OH-Gruppen in den Verbindungen (X) bzw. (VIII) durch Veretherung in die Verbindung (I) übergeführt werden, wobei $R^3$ die Bedeutung von $R^1$ hat und in $R^1$ k = 2 ist und die dem S benachbarte $-CH_2$-Gruppe nicht durch -O- oder -S- ersetzt sein darf;
bzw. ein Verfahren zur Herstellung der Verbindung der allgemeinen Formel (I), bei der $R^1$ eine Alkyloxy-Teilstruktur aufweist, das dadurch gekennzeichnet ist, daß ein 5-(4-Hydroxy-phenyl)-2-alkylthiopyrimidin der Formel (VIII) in ein 5-(4-Hydroxyphenyl)-2-alkysulfonyl-pyrimidin der Formel (IX) übergeführt wird und (IX) durch eine nukeophile Substitutionsreaktion zur Verbindung (X) umgesetzt und schließlich durch Veretherung in eine Verbindung der Formel (I) übergeführt wird. (Die Formelbilder sind im folgenden aufgeführt).

Verbindungen der Formel (I) mit $R^1$ = n-Hexyl, $R^2$ = ($C_1$- bis $C_{10}$-n-Alkyl)oxy und m = n = O sind bekannt (H. Zaschke, Diss. B, Univ. Halle/S. 1977). Sie weisen jedoch nur $S_A$-, $S_B$- und monotrope $S_G$-Phasen auf.

Verbindungen der Formel (I) mit $R^1$ = ($C_1$- bis $C_{10}$-n-Alkyl)thio, $R^2$ = ($C_1$ bis $C_{10}$-n-Alkyl)oxy und m = n = 0 sind gleichfalls bekannt (H. Zaschke et al., Z. Chem. 17, 293 (1977)). Für sie wird nur eine $S_A$-Phase ausgewiesen.

Verbindungen der Formel (I) mit $R^1$ = n-Hexyloxy, $R^2$ = ($C_4$- bis $C_6$-n-Alkyl)oxy und m = n = O sind ebenfalls bekannt (H. Zaschke, Diss. B, Univ. Halle/S., 1977). Für diese Verbindungen wurden auch wiederum nur $S_A$-Phasen gefunden.

Entgegen dem allgemeinen Kenntnisstand - wie er z.B. dokumentiert ist in "Flüssigkristalle in Tabellen". D. Demus, (Herausgeber) VEB Deutscher Verlag für Grundstoffindustrie, Leipzig, Bd. I (1974), Bd. II (1984) - wurde nun überraschend gefunden, daß die neuen Verbindungen der Formel (I) flüssigkristalline $S_c$- bzw. $S_C^*$-Phasen aufweisen.

Dieser Befund ist besonders überraschend, weil nach den umfangreichen und systematischen Variationen der Substituenten $R^1$ und $R^2$, wie sie in den zitierten Arbeiten beschrieben sind, das Auftreten von $S_c$- oder $S_C^*$-Phasen nicht zu erwarten war. Die neuen Verbindungen besitzen zum Teil sogar einen temperaturmäßig sehr breiten und überwiegend günstig, d.h. bei relativ niedrigen Temperaturen, gelegenen $S_c$- bzw. $S_C^*$-Bereich.

Die Herstellung der bekannten Verbindungen (I) mit $R^1$ = n-Hexyl, $R^2$ = ($C_1$- bis $C_{10}$-Alkyl)oxy und m = n = O ist in der weiter oben angegebenen Literatur wie folgt beschrieben:
ein Amidiniumsalz (II) wird mit einem 1-Dimethylamino-3-dimethyliminio-2-(4-$R^2$-phenyl)-propen-(1)-perchlorat (III) zu (I) kondensiert.

Für Verbindungen der Formel (I) mit $R^1$ = ($C_1$- bis $C_{10}$-n-Alkyl)thio, $R^2$ = ($C_1$- bis $C_{10}$-n-Alkyl)oxy und m = n = O ist ein Herstellungsverfahren beschrieben, bei dem ein S-Alkylisothioharnstoffbromid (IV) mit einem 1-Dimethylamino-3-dimethyliminio-2-(4-$R^2$-phenyl)-propen-(1)-perchlorat (III) in Pyridin zur Reaktion gebracht wird.

Für Verbindungen der Formel (I) mit $R^1$ = n-Hexyloxy, $R^2$ = ($C_4$- bis $C_6$-n-Alkyl)oxy und m = n = O wird folgendes Verfahren angeführt: das 1-Dimethylamino-3-dimethyliminio-2-(4-$R^2$-phenyl)-propen-(1)-perchlorat (III) wird mit Harnstoff zum Pyrimidin-2-on (V) umgesetzt. Chlorierung von (V) zur 2-Chlorpyrimidin-Verbindung (VI) mittels Phosphoroxychlorid und schließlich $S_N$-Reaktion von (VI) mit Natriumhexanolat zu (I) sind die Folgeschritte.

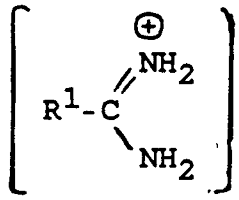

(II)

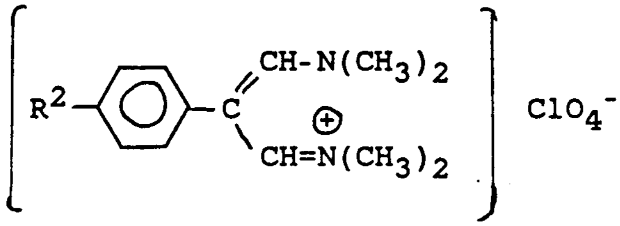

(III)

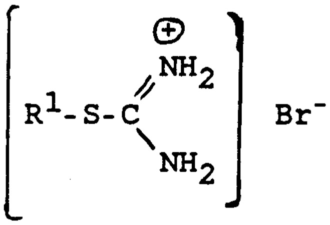

(IV)

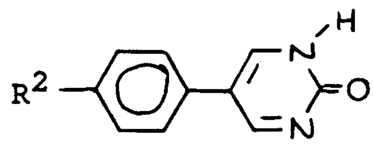

(V)

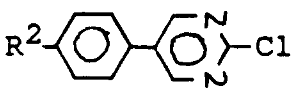

(VI)

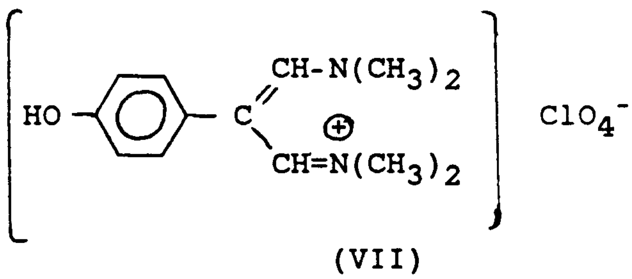

(VII)

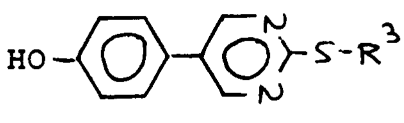

(VIII)

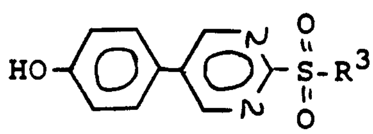

(IX)

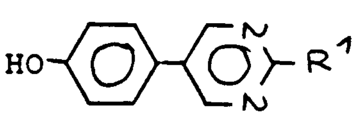

(X)

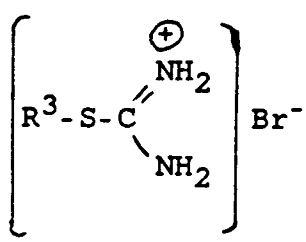

(XI)

Durch die verwendeten Ausgangsmaterialien sind bei diesen Verfahren die Substituenten $R^1$ bzw. $R^2$ schon auf der jeweils ersten Synthesestufe festgelegt. Dies gilt insbesondere für (III), bei dem Festlegung auf $R^2$ erfolgt. Breite Variation der Substituenten ist also nur mit einer entsprechenden Anzahl homologer Ausgangsmaterialien möglich.

Durch das im folgenden näher beschriebene Verfahren - das ebenfalls Gegenstand der Erfindung ist - wird dieser Nachteil behoben: durch Verwendung von (VII) für die Ringschlußreaktionen ist breite Variation von $R^2$ möglich, da die freie Hydroxygruppe in Folgereaktionen beliebig mit Verbindungen, die in

geeigneter Weise die $R^2$-Teilstruktur aufweisen, zur Reaktion gebracht werden kann. Die Synthese ist also mit einem zentralen, jedoch eine breite Variation der Substituenten, wie sie zur Erzielung optimalen Phasenverhaltens notwendig ist, gestattenden Ausgangsmaterial (VII) besonders ökonomisch. Ein weiterer vorteilhafter Aspekt des erfindungsgemäßen Verfahrens ist die Synthese von (VIII) und seine Verwendung als gemeinsames Ausgangsmaterial für Verbindungen der Formel (I), in denen $R^1$ eine Alkylthio- oder eine Alkyloxy-Teilstruktur enthält; neben breiter Variation von $R^1$ ist zusätzlich noch $R^2$ frei wählbar.

Nach dem erfindungsgemäßen, im folgenden näher erläuterten Verfahren lassen sich die Verbindungen der Formel (I) in besonders vorteilhafter Weise mit den Variationsmöglichkeiten von $R^1$ und $R^2$ herstellen, wie sie zur Erzielung optimaler Flüssigkristall-Eigenschaften, z.B. Breite und Lage der $S_c$-Phase, erforderlich sind.

Das Verfahren ist dadurch gekennzeichnet, daß ein 1-Dimethylamino-3-dimethyliminio-2-(4-hydroxy-phenyl)-propen-(1)-perchlorat (VII) - hergestellt nach literaturbekannten Standardverfahren - mit geeigneten Reaktionspartnern umgesetzt wird. So wird (VII) mit einem S-Alkylisothioharnstoffbromid (XI) zu (VIII) umgesetzt. $R^3$ hat die Bedeutung von $R^1$, $R^2$ mit k = 2 und die dem S in den allgemeinen Formeln VIII, IX und XI benachbarte $-CH_2$-Gruppe darf nicht durch -O- oder -S- ersetzt sein.

Durch Veretherung der OH-Gruppe nach literaturbekannten Verfahren mit einer Verbindung, die den Rest $R^2$ in geeigneter Weise beinhaltet, sind aus (VIII) die Verbindungen (I) erhältlich, bei denen $R^1$ eine Alkylthio-Teilstruktur aufweist.

Durch Überführung von (VIII) in das Sulfon (IX), die mit Oxidationsmitteln nach literaturbekannten Methoden erfolgen kann, und dessen weitere Umsetzung mit Verbindungen, die den Rest $R^1$ in geeigneter Weise beinhalten, sind unter nucleophiler Substitution der $-SO_2-R^3$-Teilstruktur die Verbindungen (X) erhältlich; bevorzugt wird als $R^1$-beinhaltende Verbindung ein Alkali-alkoholat eingesetzt.

Analog der Umsetzung von (VIII) zu (I) sind dann aus (X) mit einem $R^2$-Fragment auch die Verbindungen (I) erhältlich, bei denen $R^1$ eine Alkyloxy-Teilstruktur aufweist.

Durch Reaktion von (VII) mit den Amidiniumsalzen (II) zu den Verbindungen (X) und Reaktion letzterer durch Veretherung der freien OH-Gruppe mit einer Verbindung, die in geeigneter Weise den Rest $R^2$ beinhaltet, sind die Verbindungen (I) erhältlich, bei denen $R^1$ eine Alkyl-Teilstruktur aufweist.

Die folgenden Beispiele sollen die Erfindung näher erläutern:

Beispiele 1-32

2-(Hexylthio)-5-(4-hydroxy-phenyl)-pyrimidin
(VIII, $R^3 = C_6H_{13}$)

Eine Lösung von 812 g (2,55 mol) (VII) und 1339 g (5,55 mol) (XI, $R^3 = C_6H_{13}$) in 2 l Pyridin wird 6 h bei 80 °C gerührt. Nach 12-stündigem Stehen bei 25 °C wird auf Eis/Schwefelsäure gegossen; die resultierende Mischung mit pH-Wert 3 wird mit Dichlormethan extrahiert und der Extrakt über $SiO_2$ mit Dichlormethan/Methanol 99 : 1 chromatographiert. Das ölig anfallende Produkt wird mit n-Hexan kristallisiert: 458 g (62 % d.Th.) Kristalle vom Schmp. 70-72 °C.

(S)-2-Heptylthio-5-[4-(6-methyloctyloxy)phenyl]-pyrimidin

Eine Lösung von 3,0 g (0,01 mol) (VIII, $R^3 = C_7H_{15}$) in 20 ml Dimethylformamid wird mit 0,45 g (0,015 mol) Natriumhydrid (80 %) versetzt sowie nach Ende der Gasentwicklung mit 3,1 g (0,015 mol) (S)-6-Methyloctylbromid. Nach 8 h wird auf 200 ml $H_2O$ gegossen, mit Dichlormethan extrahiert und der Extrakt über $SiO_2$ mit Dichlormethan/Methanol 97 : 3 chromatographiert. Nach Umkristallisation aus n-Hexan resultieren 2 g (46 % d.Th.) farblose Kristalle; $[\alpha]_D^{25}$ : + 3,76 (c = 5, $CH_2Cl_2$)

K 44,6 $S_C^*$ 52,5 $S_A$ 59,8 I

Analog werden erhalten

(S)-2-Hexylthio-5-[4-(6-methyloctyloxy)phenyl]-pyrimidin
$[\alpha]_D^{25}$ : + 3,8 (c = 5, $CH_2Cl_2$)
K 46,8 $S_C^*$ 51,3 $S_A$62,5 I

(S)-2-Octylthio-5-[4-(6-methyloctyloxy)phenyl]-pyrimidin
$[\alpha]_D^{25}$ : + 3,52 (c = 5; $CH_2Cl_2$)
K 28,3 $S_C^*$ 55,4 $S_A$60I

(R,S)-2-Octylthio-5-[4-(4-methylhexyloxy)phenyl]-pyrimidin
K 24 $S_c$36$S_A$49,8 I

(S)-2-Nonylthio-5-[4-(6-methyloctyloxy)phenyl]-pyrimidin

$[\alpha]_D^{25}$ : + 3,4 (c = 5, $CH_2Cl_2$)
K 28,1 $S_C^*$ 53,5 $S_A$60,5 I
(S)-2-Decylthio-5-[4-(6-methyloctyloxy)phenyl]-pyrimidin
$[\alpha]_D^{25}$ : + 1,8 (c = 5, $CH_2Cl_2$)
K 35,7 $S_C^*$ 54,6 $S_A$59,9 I
(S)-2-Undecylthio-5-[4-(6-methyloctyloxy)phenyl]-pyrimidin
$[\alpha]_D^{25}$ : + 3,6 (c = 5, $CH_2Cl_2$)
K 30,8 $S_C^*$ 50,5 $S_A$56,7 I
(S)-2-Dodecylthio-5-[4-(6-methyloctyloxy)phenyl-pyrimidin
$[\alpha]_D^{25}$ : + 3,16 (c = 5, $CH_2Cl_2$)
K 39,5 $S_C^*$ 52 $S_A$57 I
(S)-2-Octylthio-5-[4-(7-methylnonyloxy)phenyl]-pyrimidin
$[\alpha]_D^{25}$ : + 3,16 (c = 5, $CH_2Cl_2$)
K 45,2 $S_C^*$ 55,2 $S_A$59,4 I
(S)-2-Octylthio-5-[4-(8-methyldecyloxy)phenyl]-pyrimidin
$[\alpha]_D^{25}$ : + 3,08 (c = 5, $CH_2Cl_2$)
K 55,4 $S_C^*$ 63 $S_A$65,3 I
(S)-2-(6-Methyloctylthio)-5-(4-octyloxyphenyl)-pyrimidin
$[\alpha]_D^{25}$ : + 4,2 (c = 5, $CH_2Cl_2$)
K[15$S_3$ 24,5$S_C^*$55,6$S_A$] 55,8 $S_A$ 64,3 I
(S),(S)-2-(6-Methyloctylthio)-5-[4-(6-methyloctyloxy) phenyl]-pyrimidin
$[\alpha]_D^{25}$ : + 9,0 (c = 3, $CH_2Cl_2$)
K[26$S_C^*$43,8$S_A$48] 52,9 I
2-Heptylthio-5-(4-heptyloxyphenyl)-pyrimidin
K 40,4 $S_c$ 42 $S_A$72,1 I
2-Heptylthio-5-(4-octyloxyphenyl)-pyrimidin
K[10,8 $S_G$35,5] 39,4 $S_c$ 50,7 $S_A$74,6 I
2-Octylthio-5-(4-octyloxyphenyl)-pyrimidin
K[18 $S_G$40] 51 $S_c$55$S_A$74,8 I
2-Octylthio-5-(4-nonyloxyphenyl)-pyrimidin
K[23,2 $S_G$38,1] 52 $S_c$58$S_A$75,8 I
2-Nonylthio-5-(4-octyloxyphenyl)-pyrimidin
K[31,6 $S_G$40,5] 47,6 $S_c$54,5 $S_A$ 74,1 I
2-Nonylthio-5-(4-nonyloxyphenyl)-pyrimidin
K[19,5 $S_G$38,5] 48,5 $S_c$ 57 $S_A$ 74,8 I
2-Nonylthio-5-(4-decyloxyphenyl)-pyrimidin
K[27$S_G$54]58,8$S_c$69,3 $S_A$ 75,7 I
2-Decylthio-5-(4-heptyloxyphenyl)-pyrimidin
K[32 $S_c$ 45] 52,3 $S_A$70,5 I
2-Decylthio-5-(4-octyloxyphenyl)-pyrimidin
K[24,5$S_G$42,2$S_c$] 54,8 $S_c$59,7$S_A$74 I
2-Decylthio-5-(4-nonyloxyphenyl)-pyrimidin
K[26,9$S_G$42,2$S_c$] 54,7$S_c$59,7$S_A$73,9 I
2-Decylthio-5-(4-decyloxyphenyl)-pyrimidin
K[35,3$S_G$57,8$S_c$] 62,1$S_c$71$S_A$75 I
2-Decylthio-5-(4-undecyloxyphenyl)-pyrimidin
K[42,5 $S_G$61,8$S_c$] 64,5$S_c$73,9$S_A$ 75 I
2-Decylthio-5-(4-dodecyloxyphenyl)-pyrimidin
K[48 $S_G$ 67,2] 69,2 $S_c$ 75,6 I
2-Undecylthio-5-(4-octyloxyphenyl)-pyrimidin
K[46,8$S_c$53,4] 61,4 $S_A$74,5 I
2-Undecylthio-5-(4-nonyloxyphenyl)-pyrimidin
K[50,0$S_c$54,7] 60,0$S_A$73,4 I
2-Undecylthio-5-(4-decyloxyphenyl)-pyrimidin
K[45,7$S_c$58,6] 62$S_c$70,9$S_A$74,8 I
2-Undecylthio-5-(4-undecyloxyphenyl)-pyrimidin
K[56,3$S_c$63,0] 65$S_c$74,2$S_A$74,7 I
2-Undecylthio-5-(4-dodecyloxyphenyl)-pyrimidin

K[45 $S_G$ 68] 71 $S_c$75 I
2-Dodecylthio-5-(4-dodecyloxyphenyl)-pyrimidin
K 64,2 $S_G$ 70,5 $S_c$75,2 I

Beispiele 33-40

2-(Hexylsulfonyl)-5-(4-hydroxy-phenyl)-pyrimidin
(IX, $R^3$ = $C_6H_{13}$)

Eine Lösung von 512 g (1,78 mol) (VIII, $R^3$ = $C_6H_{13}$) in 2,4 l Essigsäure wird bei 20 °C langsam mit 952 ml Wasserstoffperoxid (35 %) versetzt. Anschließend wird 5 h auf 50 °C erhitzt. Nach 12-stündigem Stehen bei 25 °C wird der Feststoff abgetrennt und getrocknet: 310 g (54 % d.Th.) farblose Kristalle vom Schmp. 134-137 °C.

2-(Nonyloxy)-5-(4-hydroxy-phenyl)-pyrimidin
(X, $R^1$ = $C_9H_{19}$)

Die Lösung von 133 g (0,92 mol) 1-Nonanol in 1,2 l Dimethylformamid wird mit 36,7 g (0,76 mol) Natriumhydrid (50 %) versetzt. Nach 1 h werden portionsweise vorsichtig 98 g (0,31 mol) (IX, $R^3$ = $C_6H_{13}$) zugegeben; unter starker Gasentwicklung steigt die Temperatur bis auf 54 °C. Nach 12-stündigem Stehen wird in 8 l $H_2O$ gegossen, der Feststoff abgetrennt, getrocknet und mit Hexan gewaschen: 49 g (51 % d.Th.) Kristalle vom Schmp. 89-90 °C.

2-Octyloxy-5-(4-octyloxyphenyl)-pyrimidin

Eine Lösung von 3 g (0,01 mol) (X, $R^1$ = $H_{17}C_8O$) in 50 ml Dimethylformamid wird mit 0,45 g (0,015 mol) Natriumhydrid (80 %) versetzt. Nach Ende der $H_2$-Entwicklung werden 3,6 g (0,015 mol) Octyljodid zugegeben. 7 Stunden später wird in 500 ml $H_2O$ gegossen, mit Dichlormethan extrahiert und der Extrakt über $SiO_2$ mit Dichlormethan chromatographiert. Nach Umkristallisation aus n-Hexan resultieren 2,2 g (54 % d.Th.) farblose Kristalle; K[54,7$S_c$70,9]75,8$S_A$99,8 I
Analog werden erhalten:
(S)-2-Nonyloxy-5-[4-(6-methyloctyloxy)phenyl]-pyrimidin
$[\alpha]_D^{25}$ : + 3,4 (c = 5, $CH_2Cl_2$)
K 77,7$S_C^*$ 79,2$S_A$84,7 I
2-Octyloxy-5-(4-nonyloxyphenyl)-pyrimidin
K[56,9$S_c$71]75,8$S_A$100 I
(S),(S)-2-(7-Methylnonyloxy)-5-[4-(6-methyloctyloxy) phenyl]-pyrimidin
$[\alpha]_D^{25}$ : + 6,4 (c = 5, $CH_2Cl_2$)
K 70[48$S_C^*$66$S_A$71]$S_A$71 I
2-Nonyloxy-5-[4-(7-methyloctyloxy)phenyl]-pyrimidin
K79$S_c$83$S_A$89 I
(S)-2-Octyloxy-5-[4-(6-methyloctyloxy)phenyl]-pyrimidin
$[\alpha]_D^{25}$ : + 4,06 (c = 5, $CH_2Cl_2$)
K72,8 $S_C^*$77,5$S_A$85,3 I

Beispiele 41-45

2-Nonyloxy-5-[4-(3,6-dioxadodecyloxy)phenyl]-pyrimidin

Zu einer abreagierten Mischung von 1,8 g (0,007 mol) Triphenylphosphin und 1,1 ml (0,007 mol) Diethylazodicarboxylat in 20 ml Tetrahydrofuran werden 2,2 g (0,007 mol) (X, $R^1$ = $H_{19}C_9O$) und 1,33 g (0,007 mol) Diethylenglykolmonohexylether gegeben. Nach 3-tägigem Stehen wird i. Vak. zur Trockne gebracht, der Rückstand in Dichlormethan aufgenommen und über $SiO_2$ mit Dichlormethan chromatographiert; nach Umkristallisation aus n-Hexan resultieren 1,6 g (50 % d.Th.) farblose Kristalle.
K[42$S_A$44 I]48 I
Analog werden erhalten:
2-Nonyloxy-5-[4-(10-undecen-1-yl)oxy-phenyl]-pyrimidin
K[62$S_3$67$S_c$81$S_A$91] 83$S_A$91 I

(S)-2-(4-Decyloxy-benzyloxy)-5-[4-(6-methyloctyloxy)phenyl]-pyrimidin
$[\alpha]_D^{25}$ : + 2,4 (c = 5, $CH_2Cl_2$)
K122$S_C^*$134 I
2-Octyloxy-5-[4-(4-decyloxyphenyl)methyloxy-phenyl]-pyrimidin
K[116,7$S_G$130,5$S_c$]133,4$S_c$150,0$S_A$157 I
2-Undecylthio-5-[4-(10-undecen-1-yl)oxy-phenyl]-pyrimidin
K56,0$S_3$56,3$S_c$66,3$S_A$68,0 I

Beispiele 46-49

2-Octyl-5-(4-hydroxy-phenyl)-pyrimidin
(X, $R^1$ = $C_8H_{17}$)

Zur Suspension von 120 g (0,376 mol) (VII) in 1 l Methanol werden 108 g (0,56 mo1) (II, $R^1$ = $C_8H_{17}$) sowie 206 ml Natriummethylat-Lösung (30 % in Methanol) gegeben. Nach 12-stündigem Erhitzen auf 65 °C wird i. Vak. zur Trockne gebracht und der Dichlormethan-lösliche Anteil des Rückstands über $SiO_2$ mit Dichlormethan chromatographiert. Die ölige, produkthaltige Fraktion kristallisiert beim Verrühren mit n-Hexan: 91 g (85 % d.Th.)
Kristalle vom Schmp. 94-96 °C.

(S)-2-Octyl-5-[4-(6-methyloctyloxy)phenyl]-pyrimidin

Eine Lösung von 1,4 g (0,005 mol) (X, $R^1$ = $C_8H_{17}$) in 10 ml Dimethylformamid wird mit 0,2 g (0,008 mol) Natriumhydrid (80 %) sowie nach Ende der Gasentwicklung mit 1,25 g (0,006 mol) (S)-6-Methyloctylbromid versetzt. Nach 8-stündigem Stehen wird in 100 ml $H_2O$ gegossen, mit Dichlormethan extrahiert und der Extrakt über $SiO_2$ mit Dichlormethan chromatographiert. Nach Umkristallisation aus Methanol resultieren 1 g (34 % d.Th.) farblose Kristalle,
$[\alpha]_D^{25}$ : + 3,73 (c = 10, $CH_2Cl_2$)

K40,2 $S_G^*$43,4 $S_C^*$57,8$S_A$72,3 I

Analog werden erhalten:
(S)-2-(6-Methyloctyl)-5-(4-octyloxyphenyl)-pyrimidin
$\alpha]_D^{25}$ : + 3,24 (c = 5, $CH_2Cl_2$)
K 35,8 $S_3$44,5 $S_C^*$ 54 $S_A$70,5 I
(S)-2-(8-Methyldecyl)-5-(4-octyloxyphenyl)-pyrimidin
$[\alpha]_D^{25}$ : + 3,04 (c= 5, $CH_2Cl_2$)
K 50 $S_3$60,4 $S_C^*$62,5 $S_A$74,2 I
Meßmethode: Versetzt man ein (nicht-chirales) Lösemittel mit einer kleinen Menge einer chiralen Verbindung, so wird die Ebene des linear polarisierten Lichts um den (charakteristischen) Winkel $\alpha$ gedreht; dieser Winkel wird wie folgt angegeben: $[\alpha]_D^T$ (c = x, LM), wobei die Symbole folgende Bedeutung haben: x = Konzentration der Lösung in g/l, LM = Lösemittel, D = 589 nm (NaD-Linie), T = Temperatur der Lösung. Der Drehwinkel wird in einem Polarimeter nach 10 cm Durchgang des Lichts bestimmt.

**Patentansprüche**
**Patentansprüche für folgende Vertragsstaaten : AT, BE, CH, DE, FR, GB, IT, LI, NL, SE**

**1.** 5-Phenylpyrimidin-Derivate der allgemeinen Formel (I)

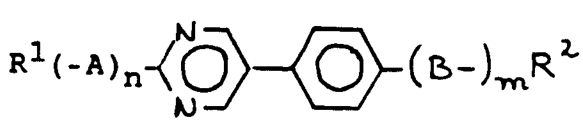

(I)

in der die Symbole folgende Bedeutung haben:

$R^1$ und $R^2$ bedeuten darin gleiche oder verschiedene, geradkettige oder verzweigte Alkyl- oder Alkenyl-Gruppen mit k bis 16 C-Atomen, mit oder ohne asymmetrisches C-Atom, worin auch eine oder mehrere nicht benachbarte $-CH_2$-Gruppen durch -O- und/oder -S- ersetzt sein können,

-A und -B bedeuten jeweils

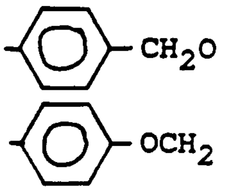

oder

wobei jeweils die Reste $R^1$, $R^2$ am Phenylenteil von -A, -B sitzen und

m und n bedeuten 0 oder 1, aber m und n nicht gleichzeitig 1, mit folgenden Maßgaben:

a) für m = n = 0 ist k = 8 und in $R^2$ ist die dem Phenylkern nächste $-CH_2$-Gruppe durch -O- ersetzt;

b) für m = 1 und n = 0 ist k = 2, und

c) für m = 0 und n = 1 ist k = 2 und in $R^2$ ist die dem Phenylkern nächste $-CH_2$-Gruppe durch -O- ersetzt.

**2.** 5-Phenylpyrimidin-Derivate der Formel I nach Anspruch 1, in der $R^1$ und $R^2$ gleiche oder verschiedene geradkettig oder verzweigte Alkyl-oder Alkenylgruppen mit 8 bis 16 C-Atomen, mit oder ohne asymetrischen C-Atom bedeuten, wobei in $R^1$ die dem Phenylkern nächste $CH_2$-Gruppe durch - S - ersetzt ist, in $R^2$ die dem Phenylkern nächste $CH_2$-Gruppe durch O ersetzt ist, und m und n 0 sind,
ausgewählt aus der Gruppe der Verbindungen
(S)-2-Heptylthio-5-(4-(6-methyloctyloxy)phenyl)-pyrimidin,
(S)-2-Octylthio-5-(4-(6-methyloctyloxy)phenyl)-pyrimidin,
(R,S)-2-Octylthio-5-(4-(4-methylhexyloxy)phenyl)-pyrimidin,
(S)-2-Nonylthio-5-(4-(6-methyloctyloxy)phenyl)-pyrimidin,
(S)-2-Decylthio-5-(4-(6-methyloctyloxy)phenyl)-pyrimidin,
(S)-2-Undecylthio-5-(4-(6-methyloctyloxy)phenyl)-pyrimidin,
(S)-2-Dodecylthio-5-(4-(6-methyloctyloxy)phenyl)-pyrimidin,
(S)-2-Octylthio-5-(4-(7-methylnonyloxy)phenyl)-pyrimidin,
(S)-2-Octylthio-5-(4-(8-methyldecyloxy)phenyl)-pyrimidin,
(S)-2-(6-Methyloctylthio)-5-(4-octyloxyphenyl)-pyrimidin,
(S),(S)-2-(6-Methyloctylthio)-5-(4-(6-methyloctyloxy)phenyl)-pyrimidin,
2-Heptylthio-5-(4-heptyloxyphenyl)-pyrimidin,
2-Heptythio-5-(4-octyloxyphenyl)-pyrimidin,
2-Octylthio-5-(4-octyloxyphenyl)-pyrimidin,
2-Octylthio-5-(4-nonyloxyphenyl)-pyrimidin,
2-Nonylthio-5-(4-octyloxyphenyl)-pyrimidin,
2-Nonylthio-5-(4-nonyloxyphenyl)-pyrimidin,
2-Nonylthio-5-(4-decyloxyphenyl)-pyrimidin,
2-Decylthio-5-(4-heptyloxyphenyl)-pyrimidin,
2-Decylthio-5-(4-octyloxyphenyl)-pyrimidin,
2-Decylthio-5-(4-nonyloxyphenyl)-pyrimidin,
2-Decylthio-5-(4-decyloxyphenyl)-pyrimidin,
2-Decylthio-5-(4-undecyloxyphenyl)-pyrimidin,
2-Decylthio-5-(4-dodecyloxyphenyl)-pyrimidin,
2-Undecylthio-5-(4-octyloxyphenyl)-pyrimidin,
2-Undecylthio-5-(4-nonyloxyphenyl)-pyrimidin,
2-Undecylthio-5-(4-decyloxyphenyl)-pyrimidin,

2-Undecylthio-5-(4-undecyloxyphenyl)-pyrimidin,
2-Undecylthio-5-(4-dodecyloxyphenyl)-pyrimidin,
2-Dodecylthio-5-(4-dodecyloryphenyl)-pyrimidin,
2-Octloxy-5-(4-octyloxyphenyl)-pyrimidin,
2-Undecylthio-5-(4-(10-undecen-1-yl)oxy-phenyl)-pyrimidin.

**3.** 5-Phenylpyrimidine der Formel I nach Anspruch 1, nämlich 2-Nonyloxy-5-(4-(10-undecen-1-yl)oxy-phenyl)-pyrimidin, (S)-2-(6-Methyloctyl)-5-(4-octyloxyphenyl)-pyrimidin.

**4.** 5-Phenylpyrimidine der Formel I nach Anspruch 1, nämlich (S)-2-(4-Decyloxy-benzyloxy)-5-(4-(6-methylocryloxy)phenyl)-pyrimidin, 2-Octyloxy-5-(4-(4-decyloxyphenyl)methyloxy-phenyl)-pyrimidin.

**5.** 5-Phenylpyrimidine der Formel I nach Anspruch 1, nämlich (S)-2-Octyl-5-(4-(6-methyloctyloxy)phenyl)-pyrimidin, (S)-2-(8-Methyldecyl)-5-(4-octyloxyphenyl)-pyrimidin.

**6.** 5-Phenylpyrimidin der Formel I nach Anspruch 1, nämlich Nonyloxy-5-(4-(3,6-dioxadodecyloxy)phenyl)-pyrimidin

**7.** Verfahren zur Herstellung einer Verbindung der allgemeinen Formel (I), dadurch gekennzeichnet, daß ein Trimethinsalz der Formel (VII) mit einem Amidiniumsalz der Formel (II) bzw. mit einem S-Alkylisothioharnstoffsalz der Formel (IV) in Gegenwart einer Base zu den Verbindungen (X) bzw. (VIII) umgesetzt wird und die freien OH-Gruppen in den Verbindungen (X) bzw. (VIII) durch Veretherung in die Verbindung (I) übergeführt werden, wobei $R^3$ die Bedeutung von $R^1$ hat und in $R^1$ und $R^2$ $k = 2$ ist und die dem S benachbarte $-CH_2$-Gruppe nicht durch -O- oder -S- ersetzt sein darf:

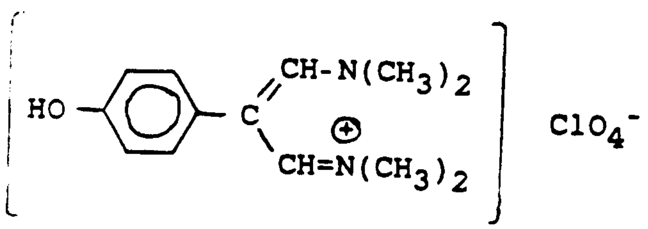

(VII)

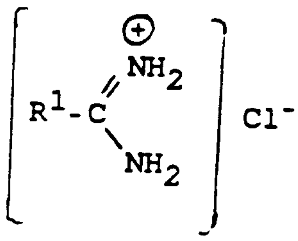

(II)

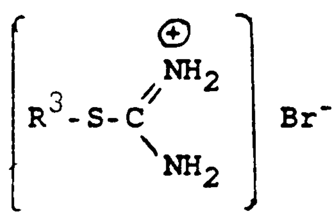

(IV)

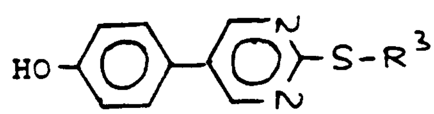

(VIII)

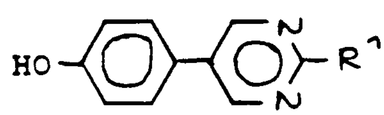

(X)

**8.** Verfahren zur Herstellung der Verbindungen der allgemeinen Formel (I), bei denen $R^1$ eine Alkyloxy-Teilstruktur aufweist, dadurch gekennzeichnet, daß ein 5-(4-Hydroxy-phenyl)-2-alkylthiopyrimidin der Formel (VIII) in ein 5-(4-Hydroxyphenyl)-2-alkylsulfonyl-pyrimidin der Formel (IX) übergeführt wird und (IX) durch eine nukleophile Substitutionsreaktion zur Verbindung (X)umgesetzt und schließlich durch Veretherung in eine Verbindung der Formel I übergeführt wird.

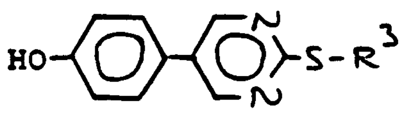 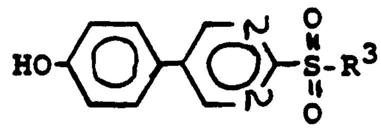

(VIII) (IX)

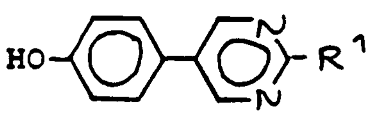 (X)

9. Verwendung der Verbindungen der allgemeinen Formel I

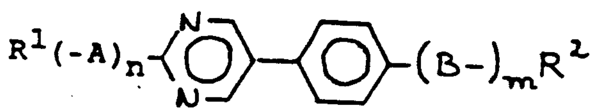 (I)

in der die Symbole folgende Bedeutung haben:

$R^1$ und $R^2$ bedeuten darin gleiche oder verschiedene, geradkettige oder verzweigte Alkyl- oder Alkenyl-Gruppen mit k bis 16 C-Atomen, mit oder ohne asymmetrisches C-Atom, worin auch ein oder mehrere nicht benachbarte $-CH_2$-Gruppen durch -0- und/oder -S- ersetzt sein können,

-A und -B bedeuten jeweils

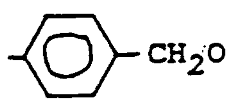 oder

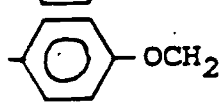

wobei jeweils die Reste $R^1$, $R^2$ am Phenylenteil von -A, -B sitzen und

m und n bedeuten 0 oder 1, aber m und n nicht gleichzeitig 1, mit folgenden Maßgaben:

a) für m = n = 0 ist k = 8 und in $R^2$ ist die dem Phenylkern nächste $-CH_2$-Gruppe durch -O- ersetzt;

b) für m = 1 und n = 0 ist k = 2, und

c) für m = 0 und n = 1 ist k = 2 und in $R^2$ ist die dem Phenylkern nächste $-CH_2$-Gruppe durch -O- ersetzt.

zur Herstellung von flüssigkristallinen Mischungen mit smektischen Phasen.

**Patentansprüche für folgenden Vertragsstaat : ES**

**1.** Verfahren zur Herstellung der 5-Phenylpyrimidin-Derivate der allgemeinen Formel I.

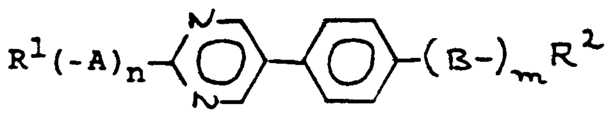 . (I)

in der die Symbole folgende Bedeutung haben:

$R^1$ und $R^2$ bedeuten darin gleiche oder verschiedene geradkettige oder verzweigte Alkyl- oder Alkenyl-Gruppen mit k bis 16 C-Atomen, mit oder ohne asymmetrisches C-Atom, worin auch eine oder mehrere nicht benachbarte $-CH_2$-Gruppen durch -O- und/oder -S- ersetzt sein können,

-A und -B bedeuten jeweils

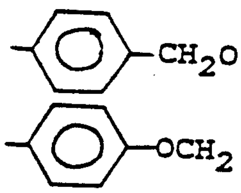 oder

wobei jeweils die Reste $R^1$, $R^2$ am Phenylenteil von -A, -B sitzen und

m und n bedeuten 0 oder 1, aber m und n nicht gleichzeitig 1, mit folgenden Maßgaben:

a) für m = n = 0 ist k = 8 und in $R^2$ ist die dem Phenylkern nächste $-CH_2$-Gruppe durch -O- ersetzt;

b) fur m = 1 und n = 0 i st k = 2, und

c) für m = 0 und n = 1 ist k = 2 und in $R^2$ ist die dem Phenylkern nächste $-CH_2$-Gruppe durch -O- ersetzt,

bei dem ein Trimethinsalz der Formel (VII) mit einem Amidiniumsalz der Formel (II) bzw. mit einem S-Alkylisothioharnstoffsalz der Formel (IV) in Gegenwart einer Base zu den Verbindungen (X) bzw. (VIII) umgesetzt wird und die freien OH-Gruppen in den Verbindungen (X) bzw. (VIII) durch Veretherung in die Verbindung (I) übergeführt werden, wobei $R^3$ die Bedeutung von $R^1$, und in $R^1$ und $R^2$ k = 2 ist und die dem S benachbarte $-CH_2$-Gruppe nicht durch -O-oder -S- ersetzt sein darf:

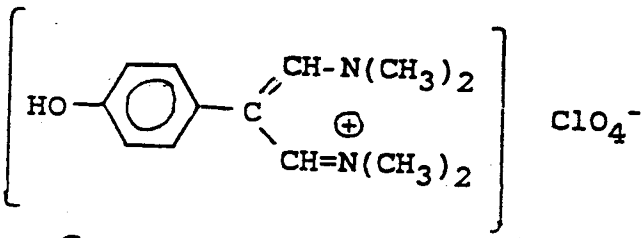

(VII)

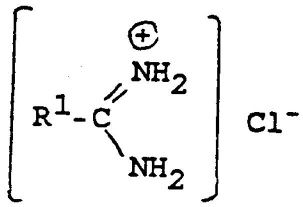

(II)

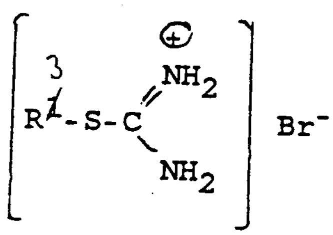

(IV)

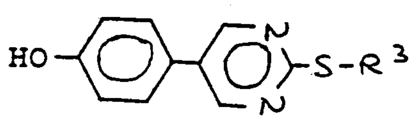

(VIII)

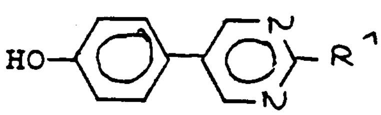

(X)

**2.** Verfahren zur Herstellung der Verbindungen der allgemeinen Formel (I) gemäß Anspruch 1, bei denen $R^1$ eine Alkyloxy-Teilstruktur aufweist, dadurch gekennzeichnet, daß ein 5-(4-Hydroxy-phenyl)-2-alkylthiopyrimidin der Formel (VIII) in ein 5-(4-Hydroxy-phenyl)-2-alkylsulfonyl-pyrimidin der Formel (IX) übergeführt wird und (IX) durch eine nucleophile Substitutionsreaktion zur Verbindung (X) umgesetzt und schließlich durch Veretherung in die Verbindung I übergeführt wird.

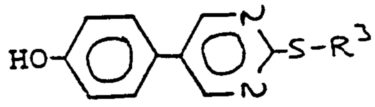

(VIII)

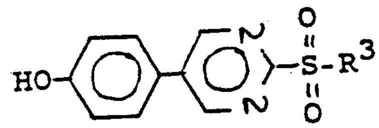

(IX)

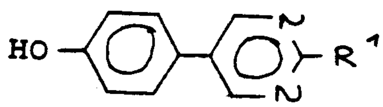

(X)

**3.** Verwendung der Verbindungen der allgemeinen Formel I

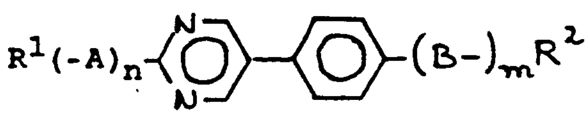

(I)

in der die Symbole folgende Bedeutung haben:

$R^1$ und $R^2$ bedeuten darin gleiche oder verschiedene, geradkettige oder verzweigte Alkyl- oder Alkenyl-Gruppen mit k bis 16 C-Atomen, mit oder ohne asymmetrisches C-Atom, worin auch ein oder mehrere nicht benachbarte $-CH_2$-Gruppen durch -0- und/oder -S- ersetzt sein können,

-A und -B bedeuten jeweils

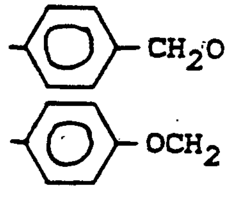

oder

wobei jeweils die Reste $R^1$, $R^2$ am Phenylenteil von -A, -B sitzen
und

m und n bedeuten 0 oder 1, aber m und n nicht gleichzeitig 1, mit folgenden Maßgaben:

a) für m = n = 0 ist k = 8 und in $R^2$ ist die dem Phenylkern nächste $-CH_2$-Gruppe durch -O- ersetzt;

b) fur m = 1 und n = 0 ist k = 2, und

c) für m = 0 und n = 1 ist k = 2 und in $R^2$ ist die dem Phenylkern nächste $-CH_2-$ Gruppe durch -O- ersetzt.

zur Herstellung von flüssigkristallinen Mischungen mit smektischen Phasen.

**Claims**

**Claims for the following Contracting States : AT, BE, CH, DE, FR, GB, IT, LI, NL, SE**

**1.** 5-Phenylpyrimidine derivatives of the general formula (I)

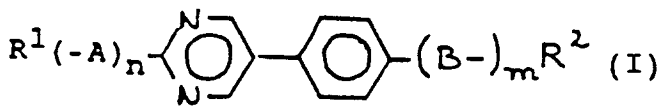

in which the symbols have the following meaning:

$R^1$ and $R^2$ denote identical or different, straight-chain or branched alkyl or alkenyl groups having from k to 16 carbon atoms, with or without an asymmetric carbon atom, in which one or more nonadjacent $-CH_2-$ groups can be replaced by -O- and/or -S-,

-A and -B each denote

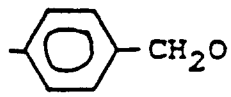

or

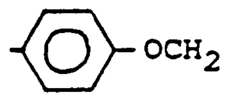

the radicals $R^1$ and $R^2$ attaching in either case to the phenylene moieties of -A and -B, and

m and n denote 0 or 1, but m and n do not simultaneously denote 1, with the following provisos:

a) if m = n = 0, k is 8, and in $R^2$ the $-CH_2-$ group adjacent to the phenyl nucleus is replaced by -O-;

b) if m = 1 and n = 0, k is 2, and

c) if m = 0 and n = 1, k is 2 and in $R^2$ the $-CH_2-$ group adjacent to the phenyl nucleus is replaced by -O-.

**2.** 5-Phenylpyrimidine derivatives of the formula I as claimed in claim 1, in which $R^1$ and $R^2$ denote identical or different, straight-chain or branched alkyl or alkenyl groups having 8 to 16 carbon atoms, with or without an asymmetric carbon atom, in which the $CH_2$ group adjacent to the phenyl nucleus is replaced by -S-, in $R^2$ the $CH_2$ group adjacent to the phenyl nucleus is replaced by O, and m and n are O,

selected from the group of compounds

(S)-2-heptylthio-5-(4-(6-methyloctyloxy)phenyl)-pyrimidine,
(S)-2-octylthio-5-(4-(6-methyloctyloxy)phenyl)-pyrimidine,
(R,S)-2-octylthio-5-(4-(methylhexyloxy)phenyl)-pyrimidine,
(S)-2-nonylthio-5-(4-(6-methyloctyloxy)phenyl)-pyrimidine,
(S)-2-decylthio-5-(4-(6-methyloctyloxy)phenyl)-pyrimidine,
(S)-2-undecylthio-5-(4-(6-methyloctyloxy)phenyl)-pyrimidine,
(S)-2-dodecylthio-5-(4-(6-methyloctyloxy)phenyl)-pyrimidine,
(S)-2-octylthio-5-(4-(7-methylnonyloxy)phenyl)-pyrimidine,
(S)-2-octylthio-5-(4-(8-methyldecyloxy)phenyl)-pyrimidine,
(S)-2-(6-methyloctylthio)-5-(4-octyloxyphenyl)-pyrimidine,
(S), (S)-2-(6-methyloctylthio)-5-(4-(6-methyloctyloxy)-phenyl)pyrimidine,
2-heptylthio-5-(4-heptyloxyphenyl)pyrimidine,
2-heptylthio-5-(4-octyloxyphenyl)pyrimidine,

2-octylthio-5-(4-octyloxyphenyl)pyrimidine,
2-octylthio-5-(4-nonyloxphenyl)pyrimidine,
2-nonylthio-5-(4-octyloxyphenyl)pyrimidine,
2-nonylthio-5-(4-nonyloxyphenyl)pyrimidine,
2-nonylthio-5-(4-decyloxyphenyl)pyrimidine,
2-decylthio-5-(4-heptyloxyphenyl)pyrimidine,
2-decylthio-5-(4-octyloxyphenyl)pyrimidine,
2-decylthio-5-(4-nonyloxyphenyl)pyrimidine,
2-decylthio-5-(4-decyloxyphenyl)pyrimidine,
2-decylthio-5-(4-undecyloxyphenyl)pyrimidine,
2-decylthio-5-(4-dodecyloxphenyl)pyrimidine,
2-undecylthio-5-(4-octyloxyphenyl)pyrimidine,
2-undecylthio-5-(4-nonyloxyphenyl)pyrimidine,
2-undecylthio-5-(4-decyloxyphenyl)pyrimidine,
2-undecylthio-5-(4-undecyloxyphenyl)pyrimidine,
2-undecylthio-5-(4-dodecyloxyphenyl)pyrimidine,
2-dodecylthio-5-(4-dodecyloxyphenyl)pyrimidine,
2-octyloxy-5-(4-octyloxyphenyl)pyrimidine,
2-undecylthio-5-(4-(10-undecen-1-yl)oxyphenyl)-pyrimidine.

**3.** 5-Phenylpyrimidines of the formula I as claimed in claim 1, namely 2-nonyloxy-5-(4-10-undecen-1-yl)-oxyphenyl)pyrimidine and (S)-2-(6-methyloctyl)5-(4-octyloxyphenyl)pyrimidine.

**4.** 5-Phenylpyrimidines of the formula I as claimed in claim 1, namely (S)-2-(4-decyloxy-benzyloxy)-5-(4-(6-methyloctyloxy)phenyl)pyrimidine and 2-octyloxy-5-(4-(4-decyloxyphenyl)methyloxyphenyl)-pyrimidine.

**5.** 5-Phenylpyrimidines of the general formula I as claimed in claim 1, namely (S)-2-octyl-5-(4-(6-methyloctyloxy)phenyl)pyrimidine and (S)-2-(8-methyldecyl)-5-(4-octyloxphenyl)pyrimidine.

**6.** 5-Phenylpyrimidines of the formula I as claimed in claim 1, namely nonyloxy-5-(4-(3,6-dioxadodecyloxy)-phenyl)pyrimidine.

**7.** A process for preparing a compound of the general formula (I), which comprises reacting a trimethine salt of the formula (VII) with an amidinium salt of the formula (II) or with an S-alkylisothiourea salt of the formula (IV) in the presence of a base to give the compounds (X) or (VIII) and converting the free OH groups in the compounds (X) or (VIII) by etherification into the compound (I) in which $R^3$ has the meaning of $R^1$ and in $R^1$ and $R^2$ k = 2 and the $-CH_2-$ group adjacent to the S must not be replaced by -O- or -S-:

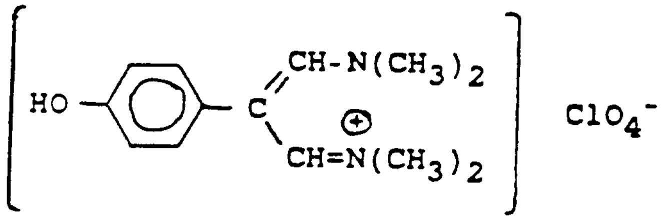

(VII)

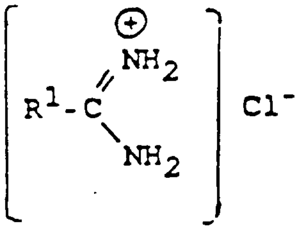

(II)

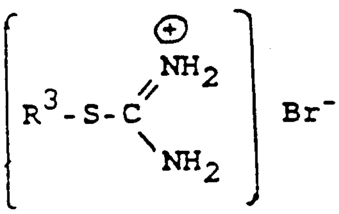

(IV)

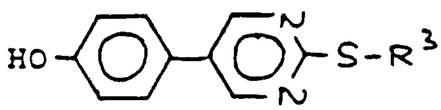

(VIII)

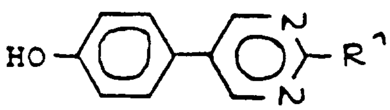

(X)

**8.** A process for preparing compounds of the general formula (I) in which $R^1$ contains an alkyloxy moiety, which comprises converting a 5-(4-hydroxyphenyl)-2-alkylthiopyrimidine of the formula (VIII) to a 5-(4-hydroxyphenyl)-2-alxylsulfonylpyrimidine of the formula (IX) and converting (IX) by a nucleophilic substitution reaction into the compound (X) and finally converting (X) by etherification into a compound of the formula I.

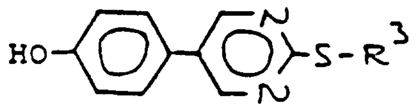

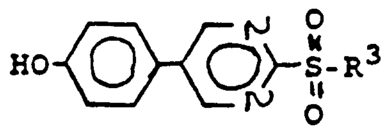

(VIII)

(IX)

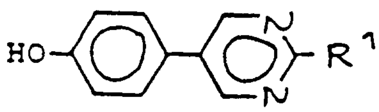

(X)

**9.** Use of the compounds of the general formula I

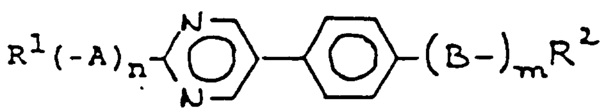

(I)

in which the symbols have the following meaning:

| | |
|---|---|
| $R^1$ and $R^2$ | denote identical or different, straight-chain or branched alkyl or alkenyl groups having from k to 16 carbon atoms, with or without an asymmetric carbon atom, in which one or more non-adjacent $-CH_2-$ groups can be replaced by -O- and/or -S-, |
| -A and -B | each denote |

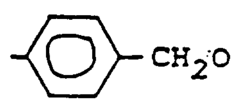

or

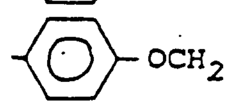

the radicals $R^1$ and $R^2$ attaching in either case to the phenylene moieties of -A and -B, and

m and n denote 0 or 1, but m and n do not simultaneously denote 1, with the following provisos:

a) if m = n = 0, k is 8, and in $R^2$ the $-CH_2-$ group adjacent to the phenyl nucleus is replaced by -O-;

b) if m = 1 and n = 0, k is 2, and

c) if m = 0 and n = 1, k is 2 and in $R^2$ the $-CH_2-$ group adjacent to the phenyl nucleus is replaced by -O-,

for preparing liquid-crystalline mixtures having smectic phases.

**Claims for the following Contracting State : ES**

**1.** A process for preparing 5-phenylpyrimidine derivatives of the general formula I

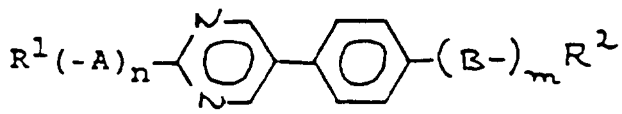 (I)

in which the symbols have the following meaning:

$R^1$ and $R^2$ denote identical or different, straight-chain or branched alkyl or alkenyl groups having k to 16 carbon atoms, with or without an asymmetric carbon atom, in which one or more non-adjacent $-CH_2-$ groups can be replaced by -O- and/or -S-,

-A and -B each denote

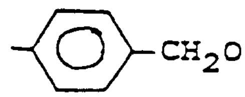 or

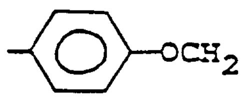

the radicals $R^1$ and $R^2$ attaching in either case to the phenylene moieties of -A and -B, and

m and n denote 0 or 1, but m and n do not simultaneously denote 1, with the following provisos:

a) if m = n = 0, k is 8, and in $R^2$ the $-CH_2-$ group adjacent to the phenyl nucleus is replaced by -O-;

b) if m = 1 and n = 0, k is 2, and

c) if m = 0 and n = 1, k is 2 and in $R^2$ the $-CH_2-$ group adjacent to the phenyl nucleus is replaced by -O-,

in which a trimethine salt of the formula (VII) is reacted with an amidinium salt of the formula (II) or with an S-alkylisothiourea salt of the formula (IV) in the presence of a base to give the compounds (X) or (VIII) and the free OH groups in the compounds (X) or (VIII) are converted by etherification into the compound (I) in which $R^3$ has the meaning of $R^1$ and in $R^1$ and $R^2$ k = 2 and the $-CH_2-$ group adjacent to the S must not be replaced by -O-or -S-:

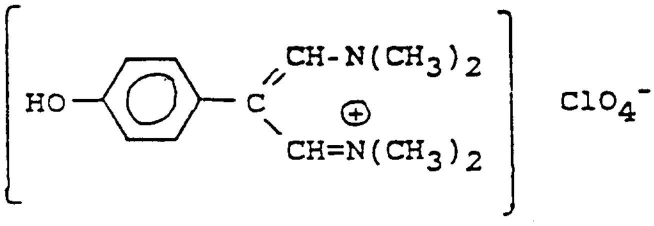

(VII)

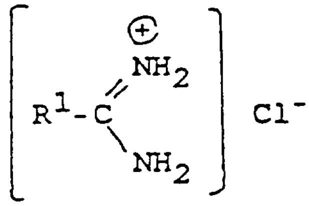

(II)

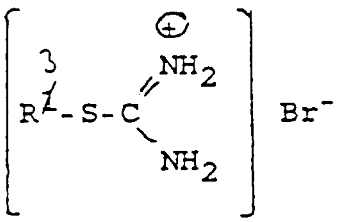

(IV)

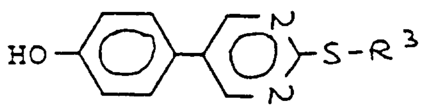

(VIII)

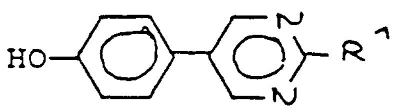

(X)

**2.** A process for preparing the compounds of the general formula (I) in which $R^1$ contains an alkyloxy moiety as claimed in claim 1, wherein a 5-(4-hydroxyphenyl)-2-alkylthiopyrimidine of the formula (VIII) is converted to a 5-(4-hydroxyphenyl)-2-alkylsulfonylpyrimidine of the formula (IX) and (IX) is converted by a nucleophilic substitution reaction into the compound (X) and finally (X) is converted by etherification into the compound I

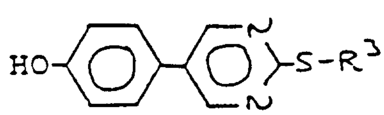

(VIII)

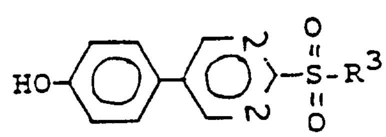

(IX)

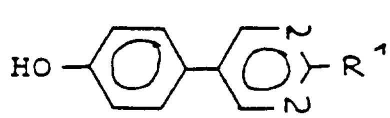

(X)

**3.** Use of the compounds of the general formula I

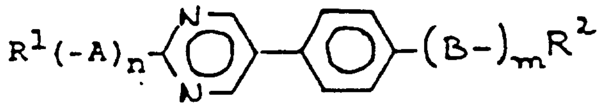 (I)

in which the symbols have the following meaning:

$R^1$ and $R^2$ denote identical or different, straightchain or branched alkyl or alkenyl groups having from k to 16 carbon atoms, with or without an asymmetric carbon atom, in which one or more nonadjacent $-CH_2-$ groups can be replaced by -O- and/or -S-,

-A and -B each denote

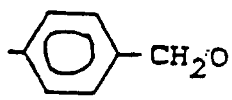 or

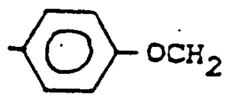

the radicals $R^1$ and $R^2$ attaching in either case to the phenylene moieties of -A and -B,

and

m and n denote 0 or 1, but m and n do not simultaneously denote 1, with the following provisos:

a) if m = n = 0, k is 8, and in $R^2$ the $-CH_2-$ group adjacent to the phenyl nucleus is replaced by -O-;

b) if m = 1 and n = 0, k is 2, and

c) if m = 0 and n = 1, k is 2 and in $R^2$ the $-CH_2-$ group adjacent to the phenyl nucleus is replaced by -O-,

for preparing liquid-crystalline mixtures having smectic phases.

**Revendications**

**Revendications pour les Etats contractant suivants : AT, BE, CH, DE, FR, GB, IT, LI, NL, SE**

**1.** Dérivés de phényl-5 pyrimidine de formule générale (I)

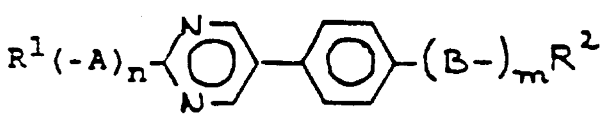 (I)

dans laquelle les symboles ont les significations suivantes:

$R^1$ et $R^2$ représentent des radicaux alkyle ou alcényle identiques ou différents, à chaîne droite ou ramifiée, ayant de k à 16 atomes de carbone, avec ou sans atome de carbone asymétrique, un ou plusieurs groupes $-CH_2-$ non voisins pouvant aussi être remplacés par -O- et/ou -S-,

-A et -B sont chacun

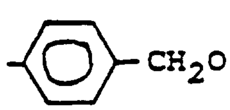 , ou

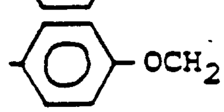

où chacun des radicaux $R^1$ et $R^2$ se trouve sur la partie phénylène de -A, -B, et

m et n valent 0 ou 1, mais m et n ne valent pas simultanément 1, avec les conditions suivantes :

a) quand m = n = 0, k = 8 et, dans $R^2$, le groupe $-CH_2-$ le plus proche du noyau phényle est remplacé par -O- ;

b) quand m = 1 et n = 0, k = 2, et

c) quand m = 0 et n = 1, k = 2 et, dans $R^2$, le groupe $-CH_2-$ le plus proche du noyau phényle est remplacépar -O-.

**2.** Dérivés de phényl-5 pyrimidine de formule I selon la revendication 1, où $R^1$ et $R^2$ sont des groupes alkyle ou alcényle identiques ou différents, à chaîne droite ou ramifiée ayant de 8 à 16 atomes de carbone, avec ou sans atome de carbone asymétrique, auquel cas dans $R^1$ le groupe $CH_2$ le plus proche du noyau phényle est remplacé par -S- et, dans $R^2$, le groupe $CH_2$ le plus proche du noyau phényle est remplacé par O, et m et n valent chacun 0,

choisis parmi l'ensemble comprenant les composés suivants :

S-heptylthio-2 ((méthyl-6 octyloxy)-4 phényl)-5 pyrimidine,
S-octylthio-2 ((méthyl-6 octyloxy)-4 phényl)-5 pyrimidine,
R,S-octylthio-2 ((méthyl-4 hexyloxy)-4 phényl)-5 pyrimidine,
S-nonylthio-2 ((méthyl-6 octyloxy)-4 phényl)-5 pyrimidine,
S-décylthio-2 ((méthyl-6 octyloxy)-4 phényl)-5 pyrimidine,
S-undécylthio-2 ((méthyl-6 octyloxy)-4 phényl)-5 pyrimidine,
S-dodécylthio-2 ((méthyl-6 octyloxy)-4 phényl)-5 pyrimidine,
S-octylthio-2 ((méthyl-7 nonyloxy)-4 phényl)-5 pyrimidine,
S-octylthio-2 ((méthyl-8 décyloxy)-4 phényl)-5 pyrimidine,
S-(méthyl-6 octylthio)-2 (octyloxy-4 phényl)-5 pyrimidine,
S,S-(méthyl-6 octylthio)-2 ((méthyl-6 octyloxy)-4 phényl)-5 pyrimidine,
Heptylthio-2 (heptyloxy-4 phényl)-5 pyrimidine,
Heptylthio-2 (octyloxy-4 phényl)-5 pyrimidine,
Octylthio-2 (octyloxy-4 phényl)-5 pyrimidine,
Octylthio-2 (nonyloxy-4 phényl)-5 pyrimidine,
Nonylthio-2 (octyloxy-4 phényl)-5 pyrimidine,
Nonylthio-2 (nonyloxy--4 phényl)-5 pyrimidine,
Nonylthio-2 (décyloxy-4 phényl)-5 pyrimidine,
Décylthio-2 (heptyloxy-4 phényl)-5 pyrimidine,
Décylthio-2 (octyloxy-4 phényl)-5 pyrimidine,
Décylthio-2 (nonyloxy-4 phényl)-5 pyrimidine,
Décylthio-2 (décyloxy-4 phényl)-5 pyrimidine,
Décylthio-2 (undécyloxy-4 phényl)-5 pyrimidine,
Décylthio-2 (dodécyloxy-4 phényl)-5 pyrimidine,
Undécylthio-2 (octyloxy-4 phényl)-5 pyrimidine,
Undécylthio-2 (nonyloxy-4 phényl)-5 pyrimidine,
Undécylthio-2 (décyloxy-4 phényl)-5 pyrimidine,
Undécylthio-2 (undécyloxy-4 phényl)-5 pyrimidine,
Undécylthio-2 (dodécyloxy-4 phényl)-5 pyrimidine,
Dodécylthio-2 (dodécyloxy-4 phényl)-5 pyrimidine,
Octyloxy-2 (octyloxy-4 phényl)-5 pyrimidine,
Undécylthio-2 ((undécène-10 yl-1)oxy-4 phényl)-5 pyrimidine

**3.** Phényl-5 pyrimidines de formule I selon la revendication 1, et plus précisément nonyloxy-2 ((undécène-10 yl-1)oxy-4 phényl)-5 pyrimidine, S-2-(méthyl-6 octyl) (octyloxy-4 phényl)-5 pyrimidine.

**4.** Phényl-5 pyrimidines de formule I selon la revendication 1, et plus précisément S-(dodécyloxy-4 benzyloxy)-2 ((méthyl-6 octyloxy)-4 phényl)-5 pyrimidine, octyloxy-2 ((décyloxy-4 phényl)-4 méthyloxy phényl)-5 pyrimidine.

**5.** Phényl-5 pyrimidines de formule I selon la revendication 1, et plus précisément S-octyl-2 ((méthyl-6 octyloxy)-4 phényl)-5 pyrimidine, S-(méthyl-8 décyl)-2 (octyloxy-4 phényl)-5 pyrimidine.

**6.** Phényl-5 pyrimidines de formule I selon la revendication 1, et plus précisément nonyloxy ((dioxa-3,6 dodécyloxy)-4 phényl)-5 pyrimidine.

**7.** Procédé pour préparer un composé de formule générale I, caractérisé en ce qu'on fait réagir un sel de triméthine de formule (VII) avec un sel d'amidinium de formule (II) ou avec un sel de S-alkylisothiourée de formule (IV) en présence d'une base, pour obtenir respectivement les composés (X) et (VIII), et on convertit les groupes OH libres des composés respectivement (X) et (VIII), par éthérification, en le composé (I), $R^3$ ayant la signification de $R^1$ et, dans $R^1$ et $R^2$, k étant égal à 2, le groupe $-CH_2-$ voisin de l'atome S ne pouvant être remplacé par -O- ou -S- :

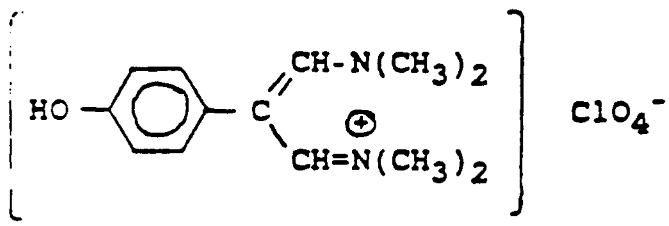

(VII)

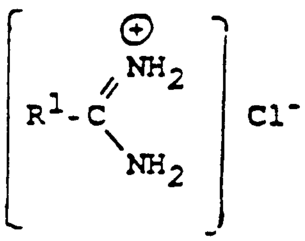

(II)

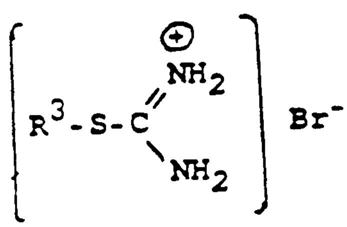

(IV)

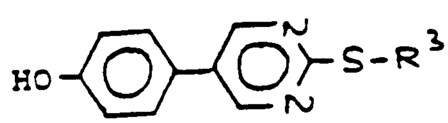

(VIII)

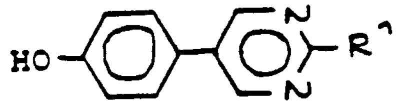

(X)

**8.** Procédé pour préparer les composés de formule générale (I), dans lequel $R^1$ a une structure partielle du type alkyloxy, caractérisé en ce qu'on convertit une (hydroxy-4 phényl)-5 alkylthio-2 pyrimidine de formule (VIII) en une (hydroxy-4 phényl)-5 alkylsulfonyl-2 pyrimidine de formule (IX), et que l'on fait réagir le composé (IX), par une réaction de substitution nucléophile, pour obtenir le composé (X), pour le convertir finalement par éthérification en un composé de formule (I).

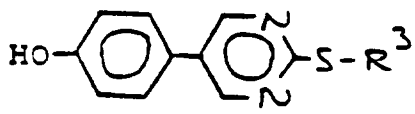

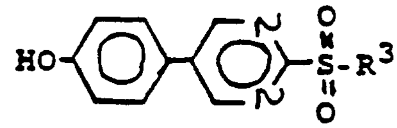

(VIII) (IX)

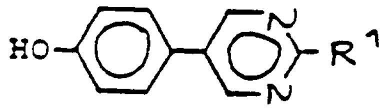

(X)

9. Utilisation des composés de formule générale I

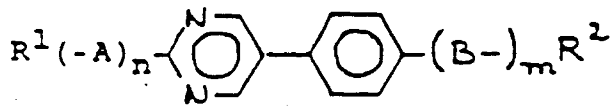

(I)

dans laquelle les symboles ont les significations suivantes:

$R^1$ et $R^2$ représentent des radicaux alkyle ou alcényle identiques ou différents, a chaîne droite ou ramifiée, ayant de k à 16 atomes de carbone, avec ou sans atomes de carbone asymétriques, un ou plusieurs groupes $-CH_2-$ non voisins pouvant aussi être remplacés par -O- et/ou -S-,

-A et -B sont chacun

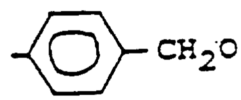

ou

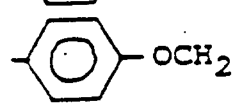

où chacun des radicaux $R^1$ et $R^2$ se trouve sur la partie phénylène de -A, -B, et

m et n valent 0 ou 1, mais m et n ne valent pas simultanément 1, avec les conditions suivantes :

a) quand m = n = 0, k = 8 et, dans $R^2$, le groupe $-CH_2$ le plus proche du noyau phényle est remplacé par -O- ;

b) quand m = 1 et n = 0, k = 2, et

c) quand m = 0 et n = 1, k = 2 et, dans $R^2$, le groupe $-CH_2-$ le plus proche du noyau phényle est remplacépar -O-,

pour préparer des mélanges mésomorphes comportant des phases smectiques.

**Revendications pour l'Etat contractant suivant : ES**

**1.** Procédé pour préparer des dérivés de phényl-5 pyrimidine de formule générale I

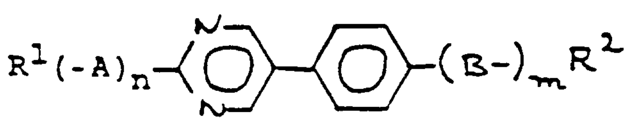

. (I)

dans laquelle les symboles ont les significations suivantes:

$R^1$ et $R^2$ représentent des radicaux alkyle ou alcényle identiques ou différents, à chaîne droite ou ramifiée, ayant de k à 16 atomes de carbone, avec ou sans atomes de carbone asymétriques, un ou plusieurs groupes $-CH_2-$ non voisins pouvant aussi être remplacés par -O- et/ou -S-,

-A et -B sont chacun

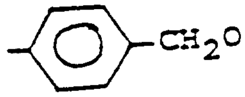

ou

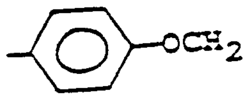

où chacun des radicaux $R^1$ et $R^2$ se trouve sur la partie phénylène de -A, -B, et

m et n valent 0 ou 1, mais m et n ne valent pas simultanément 1, avec les conditions suivantes :

a) quand m = n = 0, k = 8 et, dans $R^2$, le groupe $-CH_2-$ le plus proche du noyau phényle est remplacé par -O- ;

b) quand m = 1 et n = 0, k = 2, et

c) quand m = 0 et n = 1, k = 2 et, dans $R^2$, le groupe $-CH_2-$ le plus proche du noyau phényle est remplacé par -O-,

dans lequel on fait réagir un sel de triméthine de formule (VII) avec un sel d'amidinium de formule (II) ou avec un sel de S-alkylisothiourée de formule (IV) en présence d'une base, pour obtenir respectivement les composés (X) et (VIII), et on convertit les groupes OH libres des composés respectivement (X) et (VIII), par éthérification, en le composé (I), $R^3$ ayant la signification de $R^1$ et, dans $R^1$ et $R^2$, k étant égal à 2, le groupe $-CH_2-$ voisin de l'atome S ne pouvant être remplacé par -O- ou -S-

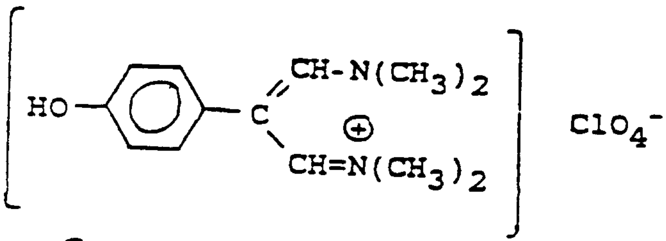

(VII)

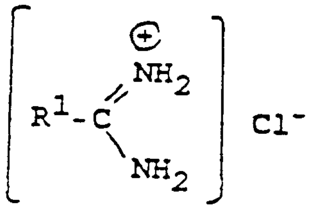

(II)

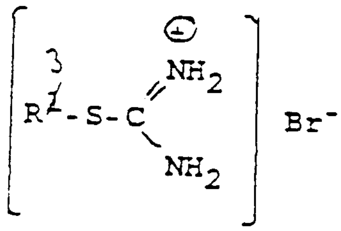

(IV)

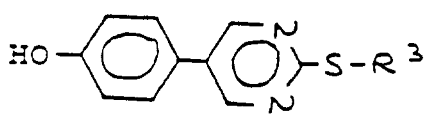

(VIII)

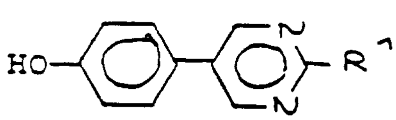

(X)

2. Procédé pour préparer les composés de formule générale (I), dans lequel $R^1$ a une structure partielle du type alkyloxy, caractérisé en ce qu'on convertit une (hydroxy-4 phényl)-5 alkylthio-2 pyrimidine de formule (VIII) en une (hydroxy-4 phényl)-5 alkylsulfonyl-2 pyrimidine de formule (IX), et que l'on fait réagir le composé (IX), par une réaction de substitution nucléophile, pour obtenir le composé (X), pour le convertir finalement par éthérification en un composé de formule (I).

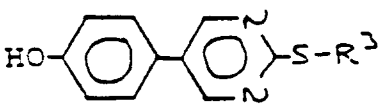

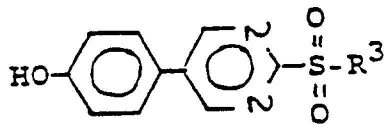

(VIII)

(IX)

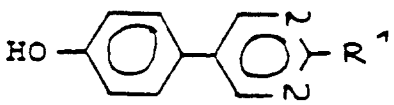

(X)

3. Utilisation des composés de formule générale I

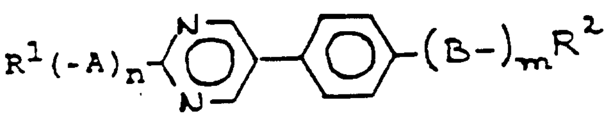

(I)

dans laquelle les symboles ont les significations suivantes:

$R^1$ et $R^2$ représentent des radicaux alkyle ou alcényle identiques ou différents, à chaîne droite ou ramifiée, ayant de k à 16 atomes de carbone, avec ou sans atomes de carbone asymètriques, un ou plusieurs groupes $-CH_2-$ non voisins pouvant aussi être remplacés par -O- et/ou -S-,

-A et -B sont chacun

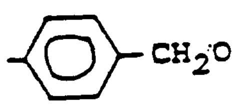

ou

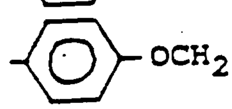

où chacun des radicaux $R^1$ et $R^2$ se trouve sur la partie phénylène de -A, -B, et

m et n valent 0 ou 1, mais m et n ne valent pas simultanément 1, avec les conditions suivantes :

a) quand m = n = 0, k = 8 et, dans $R^2$, le groupe $-CH_2-$ le plus proche du noyau phényle est remplacé par -O- ;

b) quand m = 1 et n = 0, k = 2, et

c) quand m = 0 et n = 1, k = 2 et, dans $R^2$, le groupe $-CH_2-$ le plus proche du noyau phényle est remplacé par -O-,

pour préparer des mélanges mésomorphes comportant des phases smectiques.